# EUROPEAN PATENT APPLICATION

(11) **EP 4 238 962 A1**
(43) Date of publication of application: **06.09.2023**
(21) Application number: 21885882.7
(22) Date of filing: 12.10.2021
(51) Int. Cl.: C07D 209/44, C07D 471/04, C07D 487/04, C07D 495/04

(54) **METHOD FOR PRODUCING DIIMINOPYRROLE COMPOUND**

(30) Priority: 30.10.2020 JP 2020182459
(71) Applicant: Godo Shigen Co., Ltd., Chiba, 299-4333 (JP)
(72) Inventor: MIYAMOTO Michihiko, Chosei-gun, Chiba 299-4333 (JP); KOMATSU Hiroto, Chosei-gun, Chiba 299-4333 (JP); YAMADA Shinichiro, Chosei-gun, Chiba 299-4333 (JP); JITSUKAWA Takuya, Chosei-gun, Chiba 299-4333 (JP); TAGAMI Koichi, Chosei-gun, Chiba 299-4333 (JP); YAMAMOTO Takahiro, Chosei-gun, Chiba 299-4333 (JP); TOGO Hideo, Chiba-shi, Chiba 263-8522 (JP)
(74) Representative: Rings, Rolf
(86) International application number: PCT/JP2021/037652
(87) International publication number: WO 2022/091763

(57) **Abstract**

[Problem]

Provided is a method for producing a diiminopyrrole compound suitable for improving producibility and eventually reducing a production cost by actively utilizing iodine, which is a resource that is mass-producible domestically in Japan.

[Solution]

A method for producing a diiminopyrrole compound includes producing a diiminopyrrole compound by mixing an aromatic 3-pyrroline compound, an iodinating agent, and ammonia based on Chemical Formula 1 below;

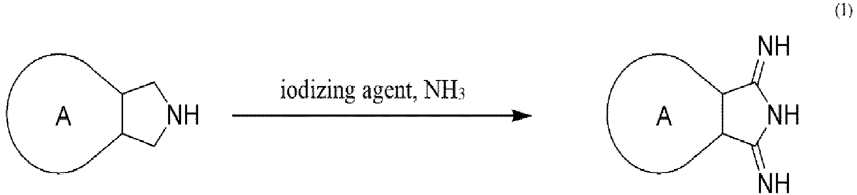

Here, a ring A is an aromatic group.

## Description

### TECHNICAL FIELD

The present invention relates to a method for producing a diiminopyrrole compound, in particular, relates to a method for producing a diiminopyrrole compound suitable for improving producibility and eventually reducing a production cost by actively utilizing iodine, which is a resource that is mass-producible domestically in Japan.

### BACKGROUND ART

A diiminopyrrole compound is a compound for a raw material of a phthalocyanine dye, which is widely spread as an organic pigment. Phthalocyanine pigments have colors between blue and green, are very vivid with significant pigmenting power, and are stable with respect to light, heat, temperature, and the like thus being excellent in toughness. Moreover, this phthalocyanine pigment is an almost ideal pigment as it can be produced at a relatively low cost, and has been used in a wide range of fields including printing inks, coating materials, plastics, writing materials, printing, and the like.

The diiminopyrrole compound is industrially produced using phthalonitrile or phthalic anhydride as a raw material. However, production methods of these raw materials need to use a cyanide, which is highly toxic, and a strong oxidant, and therefore they have a large impact on the environment thus posing a problem for safety. In view of this, there has been desired a safe method for producing a diiminopyrrole compound without undergoing these raw materials, but such a technique has not been specifically proposed conventionally.

Note that, in a method for producing a diiminoisoindoline compound, which is a kind of diiminopyrrole compound that uses this phthalonitrile as a raw material, there has been proposed a method that causes ammonia to react in the presence of sulfur as shown in Formula (3) below (for example, see Non-Patent Document 1).

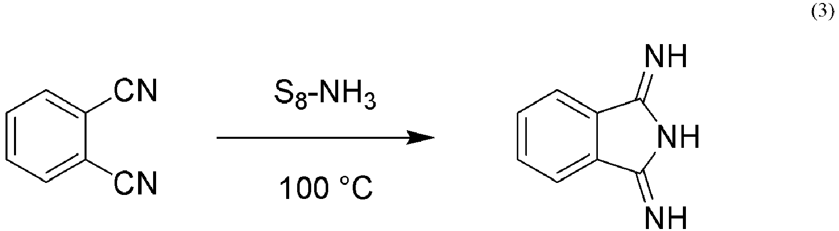

In a producing method using a phthalic anhydride derivative as a raw material, as shown in Formula (4) below, there has been proposed a method for producing a diiminoisoindoline compound by causing the phthalic anhydride derivative to react with urea and ammonium nitrate at 60°C to 160°C in a presence of a molybdic acid catalyst and being added with a caustic alkali after being cooled (for example, see Patent Document 1).

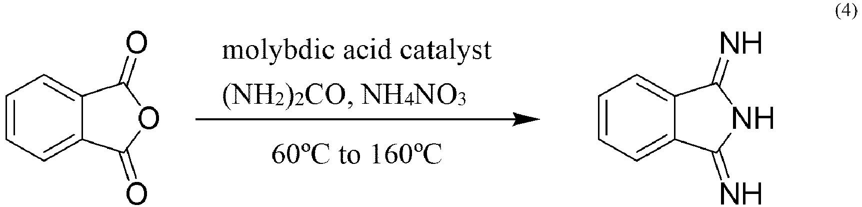

Patent Document 1: JP-A-H07-330729

Non-Patent Document 1: Chem. Lett. 1984, 1423 to 1426.

### DISCLOSURE OF THE INVENTION

### PROBLEMS TO BE SOLVED BY THE INVENTION

The disclosed technique of Non-Patent Document 1 described above sometimes causes an increased production cost as the phthalonitrile as the used raw material is expensive. The disclosed technique of Patent Document 1 described above is a producing method that gives an impact on the environment as a heavy metal catalyst (the molybdic acid catalyst) is necessary for producing the diiminoisoindoline compound.

Therefore, the present invention has been invented in consideration of the above-described problems, and an object thereof relates to a method for producing a diiminopyrrole compound, and relates to a method for producing a diiminopyrrole compound suitable for improving producibility and eventually reducing a production cost by using iodine and ammonia, which are environmentally friendly.

### SOLUTIONS TO THE PROBLEMS

In order to solve the above-described problems, the inventors have newly found that, by mixing an iodinating agent, which is mass-producible at a low price, and ammonia into an aromatic 3-pyrroline compound, a diiminopyrrole compound can be produced.

A method for producing a diiminopyrrole compound according to a first invention includes producing a diiminopyrrole compound by mixing an aromatic 3-pyrroline compound, an iodinating agent, and ammonia based on Chemical Formula 1 below.

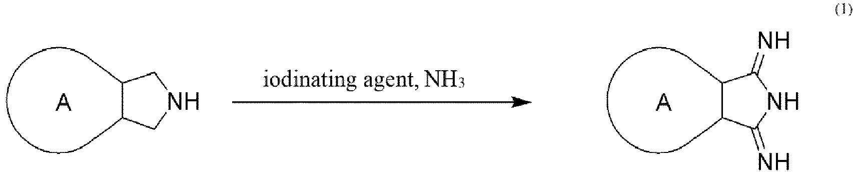

Here, a ring A is an aromatic group.

The method for producing a diiminopyrrole compound according to a second invention, which is in the first invention, when producing the diiminopyrrole compound based on Chemical Formula 1, the ring A is an aromatic group having heteroatoms.

The method for producing a diiminopyrrole compound according to a third invention, which is in the second invention, when producing the diiminopyrrole compound based on Chemical Formula 1, the ring A is any one of a six-membered aromatic group having heteroatoms, a five-membered aromatic group having heteroatoms, and a bicyclic aromatic group having heteroatoms.

The method for producing a diiminopyrrole compound according to a fourth invention, which is in the first invention, when producing the diiminopyrrole compound based on Chemical Formula 1, the ring A is an aromatic group without heteroatoms.

A method for producing a diiminopyrrole compound according to a fifth invention includes producing a diiminopyrrole compound by mixing a precursor made of an o-bis(halogenomethylene) aromatic compound , an iodinating agent, and ammonia based on Chemical Formula 2 below.

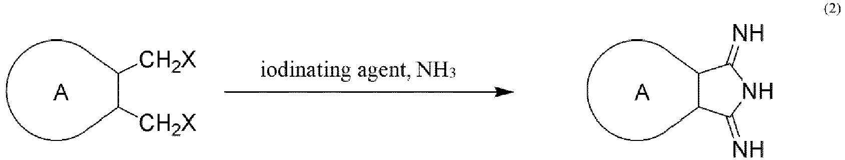

Here, a ring A is an aromatic group and X is halogen (I, Br, Cl).

The method for producing a diiminopyrrole compound according to a sixth invention, which is in the fifth invention, when producing the diiminopyrrole compound based on Chemical Formula 2, the ring A is an aromatic group having heteroatoms.

The method for producing a diiminopyrrole compound according to a seventh invention, which is in the sixth invention, when producing the diiminopyrrole compound based on Chemical Formula 2, the ring A is any one of a six-membered aromatic group having heteroatoms, a five-membered aromatic group having heteroatoms, and a bicyclic aromatic group having heteroatoms.

The method for producing a diiminopyrrole compound according to an eighth invention, which is in the fifth invention, when producing the diiminopyrrole compound based on Chemical Formula 2, the ring A is an aromatic group without heteroatoms.

### EFFECTS OF THE INVENTION

The present invention formed of the above-described configurations ensures producing the diiminopyrrole compound by using the aromatic 3-pyrroline compound, which is easily available, as a starting material and processing the aromatic 3-pyrroline compound with the iodinating agent and the ammonia, which are environmentally friendly, and thus, producibility is improved and eventually, a production cost is reducible.

The present invention formed of the above-described configurations is a producing method performed under a gentle condition where a strong oxidant or the like is not used and ensures synthesizing a diiminopyrrole compound having unstable heteroatoms so as to ensure a conversion into high-functional heterocyclic phthalocyanine, which is expected to have various functions.

### DESCRIPTION OF PREFERRED EMBODIMENTS

The following describes methods for producing a diiminopyrrole compound according to the embodiments of the present invention in detail.

A method for producing a diiminopyrrole compound to which the present invention is applied mixes an aromatic 3-pyrroline compound, an iodinating agent, and ammonia in a solvent based on Chemical Formula 1 below.

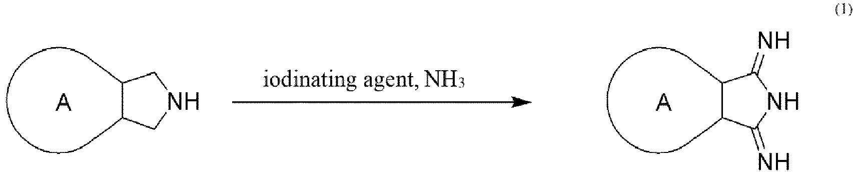

Here, a ring A is any one of 1) an aromatic group, 2) an aromatic group having heteroatoms, 3) a six-membered aromatic group having heteroatoms, 4) a five-membered aromatic group having heteroatoms, 5) a bicyclic aromatic group having heteroatoms, and 6) an aromatic group without heteroatoms.
Here, 1) the aromatic group is an aromatic hydrocarbon ring group or an aromatic heterocyclic group and is specifically, a phenyl group, a biphenylyl group, a terphenylyl group, a naphthyl group, a binaphthylyl group, an azulenyl group, an anthracenyl group, a phenanthrenyl group, a furyl group, a thienyl group, a pyrrolyl group, a pyrazolyl group, an imidazolyl group, an isoxazolyl group, a thiazolyl group, a thiadiazolyl group, a pyridyl group, a pyrazinyl group, a pyrimidinyl group, a pyridazinyl group, a benzofuryl group, an indolyl group, a quinolyl group, an isoquinolyl group, a quinazolinyl group, a quinoxalinyl group, a cinnolinyl group, a benzothiazolyl group, a carbazolyl group, a phenanthridinyl group, a phenazinyl group, a phenoxazinyl group, a phenothiazinyl group, or the like.
   This aromatic group may be substituted, and the number of substituents in such a case is one or plural, which means it is not specifically limited as long as they can be substituted. The number of substituents in the aromatic group that may be substituted is one or plural, which means it is not specifically limited as long as they can be substituted, and examples of the groups that may be substituted include halogen atoms, a linear or branched-chain alkyl group with 1 to 12 carbon atoms that may be substituted, an aromatic group that may be substituted, a non-aromatic heterocyclic group that may be substituted, a carboxyl group, a linear or branched-chain alkoxy group with 1 to 12 carbon atoms, a cyano group, a nitro group, and the like.
2) The aromatic group having heteroatoms includes, besides all of 3) to 5), a carbazolyl group (tricyclic), a phenanthridinyl group (tricyclic), a phenazinyl group (tricyclic), a phenoxazinyl group (tricyclic), and a phenothiazinyl group (tricyclic). The aromatic group having heteroatoms is not limited to the configurations of 3) to 5), and includes any aromatic group as long as it has heteroatoms. The heteroatom in the aromatic group having heteroatoms is, for example, nitrogen, oxygen, and sulfur.
3) The six-membered aromatic group having heteroatoms is, for example, a pyridyl group, a pyrazinyl group, a pyrimidinyl group, and a pyridazinyl group.
4) The five-membered aromatic group having heteroatoms is, for example, a furyl group, a thienyl group, a pyrrolyl group, a pyrazolyl group, an imidazolyl group, an isoxazolyl group, a thiazolyl group, and a thiadiazolyl group.
5) The bicyclic aromatic group having heteroatoms is, for example, a benzofuryl group, an indolyl group, a quinolyl group, an isoquinolyl group, a quinazolinyl group, a quinoxalinyl group, a cinnolinyl group, and a benzothiazolyl group.
6) The aromatic group without heteroatoms is, for example, a phenyl group, a biphenylyl group, a terphenylyl group, a naphthyl group, a binaphthyl group, an azulenyl group, an anthracenyl group, and a phenanthrenyl group.

The iodinating agent is, for example, iodine, iodine monochloride, N-Iodosuccinimide (NIS), and 1,3-Diiodo-5,5-dimethylhydantoin (DIH).

The reaction solvent may be solvent-free or any solvent as long as it does not interfere with an iodination reaction with the iodinating agent. The reaction solvent is preferably a solvent used in a common iodination reaction, and is, for example, a halogen-type solvent, such as dichloroethane and chloroform, an ether-type solvent, such as tetrahydrofuran and diethyl ether, a hydrocarbon-type solvent, such as toluene and benzene, a protic polar solvent, such as methanol, ethanol, and water, and an aprotic polar solvent, such as acetonitrile and acetone.

The synthesis condition in the method for producing the diiminopyrrole compound to which the present invention is applied is to mix the iodinating agent and the ammonia with the aromatic 3-pyrroline compound. Approximately 1 to 10 equivalents of the iodinating agent is mixed with the aromatic 3-pyrroline compound as the starting material. Approximately 4 to 6 equivalents of this iodinating agent is preferably mixed with the aromatic 3-pyrroline compound as the starting material. The ammonia is made of, for example, a liquid in which an ammonia water, an ammonia gas, or ammonia is dissolved by an organic solvent. Approximately 1 to 200 equivalents of the ammonia is mixed with the aromatic 3-pyrroline compound as the starting material. Approximately 20 to 60 equivalents of this ammonia is preferably mixed with the aromatic 3-pyrroline compound as the starting material.

The reaction temperature is usually a temperature at a boiling point or less of the solvent and is preferably 0°C to 100°C, more preferably, a room temperature to 60°C. The reaction time is usually 15 minutes to 24 hours.

Note that, the present invention may be configured to mix a precursor of the aromatic 3-pyrroline compound, the iodinating agent, and the ammonia as shown in Formula 2 below. This precursor is made of an o-bis(halogenomethylene) aromatic compound .

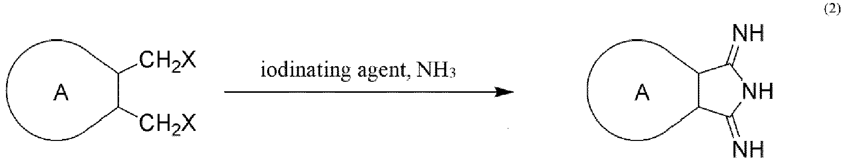

Here, a ring A is similar to that in Formula 1, and X is halogen (I, Br, Cl).

For the synthesis condition of Formula 2, the iodinating agent and the ammonia are mixed with the precursor. Approximately 1 to 10 equivalents of the iodinating agent is mixed with the precursor as the starting material. Approximately 4 to 6 equivalents of this iodinating agent is preferably mixed with the precursor as the starting material. Approximately 1 to 200 equivalents of the ammonia is mixed with the precursor as the starting material. Approximately 20 to 60 equivalents of this ammonia is preferably mixed with the precursor as the starting material.

### [Examples]

An aromatic 3-pyrroline compound (0.5 mmol) was added to an organic solvent (2 mL). Next, under an ice-cold condition, a 28% ammonia water (1 mL) and an iodinating agent (5 equiv.) were added and stirred for four hours at room temperature. After the reaction ended, a sodium thiosulfate solution was added and extracted with butanol (10 mL) three times. After drying the resulting organic layer with sodium sulfate, the solvent was distilled away, and thus, a diiminopyrrole compound was obtained. Each yield is shown in Table 1 below.

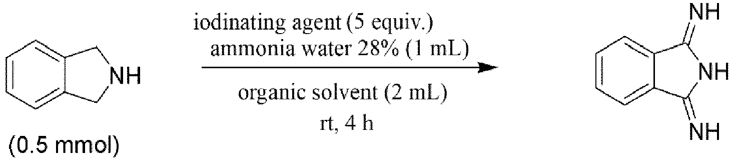

| example | iodinating agent | organic solvent | yield |
|---|---|---|---|
| 1 | NIS¹⁾ | CH₂Cl₂ | 66% |
| 2 | DIH²⁾ | CH₂Cl₂ | 61% |
| 3 | I₂ | CH₂Cl₂ | 85% |
| 4 | I₂ | none | 52% |
| 5 | I₂ | toluene | 57% |
| 6 | I₂ | CH₃OH | 33% |
| 7 | I₂ | CH₃CN | 51% |
| 8 | I₂ | THF | 41% |
| 9 | I₂ | DMF | 22% |

| | | | |
|---|---|---|---|
| 1) NIS: N-Iodosuccinimide 2) DIH: 1,3-Diiodo-5,5-dimethylhydantoin | | | |

Each aromatic 3-pyrroline compound (0.5 mmol) was added to dichloromethane (2 mL). Next, under an ice-cold condition, a 28% ammonia water (1 mL) and an iodine (5 equiv.) were added and stirred for four hours at room temperature. After the reaction ended, a sodium thiosulfate solution was added and extracted with butanol (10 mL) three times. After drying the resulting organic layer with sodium sulfate, the solvent was distilled away, and thus, a diiminopyrrole compound was obtained. Each yield is shown in Table 2 below.

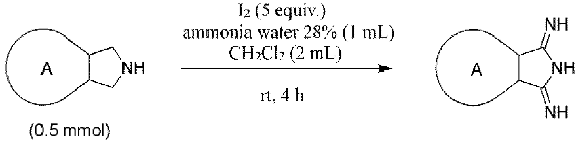

| example | aromatic 3-pyrroline compound | product | yield |
|---|---|---|---|
| 10 | 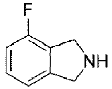 | 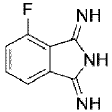 | 59% |
| 11 | 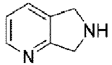 | 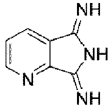 | 46% |
| 12 | 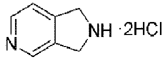 | 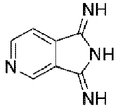 | 51% |
| 13 | 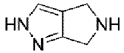 | 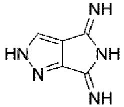 | 38% |

The following shows the ¹H NMR measurement results of the respective products of Examples 10 and 13. The product of Example 10, 1,3-Diimino-4-fluoro-1H-isoindol: ¹H-NMR (400 MHz, DMSO-d6): δ = 7.36 (t, 1H), 7.57-7.64 (m, 1H), 7.74 (d, 1H), 8.73 (br s, 3H)
The product of Example 13, Pyrrolo[3,4-c]pyrazole-4,6-diimine: ¹H-NMR (400 MHz, DMSO-d6): δ = 7.84 (s, 1H)

An o-bis(halogenomethylene) aromatic compound (0.5 mmol) and iodine (5 equiv.) were added to a 28% ammonia water (1 mL) and stirred for four hours at a predetermined temperature. After the reaction ended, a sodium thiosulfate solution was added and extracted with butanol (10 mL) three times. After drying the resulting organic layer with sodium sulfate, the solvent was distilled away, and thus, a diiminopyrrole compound was obtained. Each yield is shown in Table 3 below.

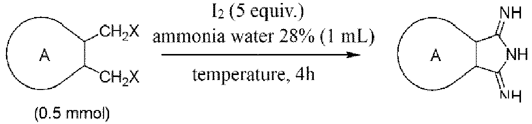

| example | o-bis(halogenomethylene) aromatic compound | temperature | product | yield |
|---|---|---|---|---|
| 14 | 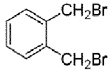 | 40°C | 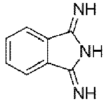 | 51% |
| 15 | 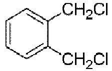 | 60°C | 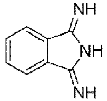 | 42% |
| 16 | 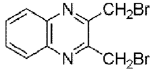 | 40°C | 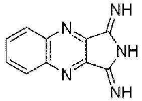 | 63% |
| 17 | 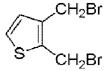 | 40°C | 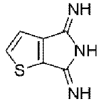 | 23% |

## Claims

1. A method for producing a diiminopyrrole compound, comprising
producing a diiminopyrrole compound by mixing an aromatic 3-pyrroline compound, an iodinating agent, and ammonia based on Chemical Formula 1 below; 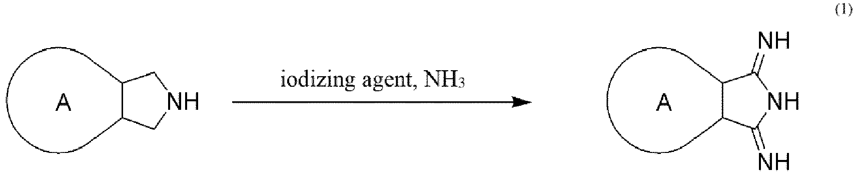 here, a ring A is an aromatic group.

2. The method for producing a diiminopyrrole compound according to claim 1, wherein
when producing the diiminopyrrole compound based on Chemical Formula 1, the ring A is an aromatic group having heteroatoms.

3. The method for producing a diiminopyrrole compound according to claim 2, wherein
when producing the diiminopyrrole compound based on Chemical Formula 1, the ring A is any one of a six-membered aromatic group having heteroatoms, a five-membered aromatic group having heteroatoms, and a bicyclic aromatic group having heteroatoms.

4. The method for producing a diiminopyrrole compound according to claim 1, wherein
when producing the diiminopyrrole compound based on Chemical Formula 1, the ring A is an aromatic group without heteroatoms.

5. A method for producing a diiminopyrrole compound, comprising
producing a diiminopyrrole compound by mixing a precursor made of an o-bis(halogenomethylene) aromatic compound , an iodinating agent, and ammonia based on Chemical Formula 2 below; 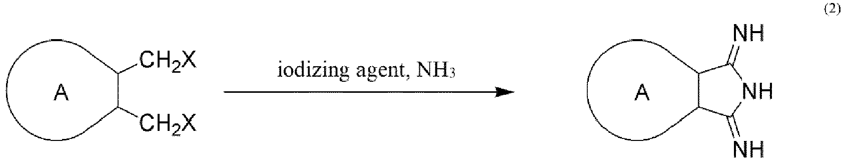 here, a ring A is an aromatic group and X is halogen (I, Br, Cl).

6. The method for producing a diiminopyrrole compound according to claim 5, wherein
when producing the diiminopyrrole compound based on Chemical Formula 2, the ring A is an aromatic group having heteroatoms.

7. The method for producing a diiminopyrrole compound according to claim 6, wherein
when producing the diiminopyrrole compound based on Chemical Formula 2, the ring A is any one of a six-membered aromatic group having heteroatoms, a five-membered aromatic group having heteroatoms, and a bicyclic aromatic group having heteroatoms.

8. The method for producing a diiminopyrrole compound according to claim 5, wherein
when producing the diiminopyrrole compound based on Chemical Formula 2, the ring A is an aromatic group without heteroatoms.
